# EUROPEAN PATENT SPECIFICATION

(11) **EP 0 593 562 B1**
(45) Date of publication and mention of the grant of the patent: **17.03.1999**
(21) Application number: 92914132.3
(22) Date of filing: 03.07.1992
(51) Int. Cl.: A61K 31/52, A61K 47/00, A61K 47/10

(54) **TOPICAL COMPOSITION CONTAINING PENCICLOVIR**
TOPISCH ANZUWENDENDES ARZNEIMITTEL ENTHALTEND PENCICLOVIR
COMPOSITION TOPIQUE CONTENANT DU PENCICLOVIR

(30) Priority: 11.07.1991 GB 9114939
(43) Date of publication of application: 27.04.1994
(73) Proprietor: SMITHKLINE BEECHAM PLC, Brentford, Middlesex TW8 9EP (GB)
(72) Inventor: GOODALL, Alan, John, Worthing West Sussex BN14 8QH (GB); GRIFFITHS, Hazel-Ann, Dorking Surrey RH4 1NE (GB); ODURO-YEBOAH, Joshua, Worthing West Sussex BN14 8QH (GB)
(74) Representative: Tocher, Pauline
(86) International application number: GB9201208
(87) International publication number: WO9300905

(56) References cited:
- EP-A- 0 416 739
- WO-A-91/11187

## Description

This invention relates to a topical pharmaceutical formulation suitable for use in the treatment of virus infections of the skin and mucosa.

EP-A-141927 (Beecham Group p.l.c.) discloses the compound 9-(4-hydroxy-3-hydroxymethylbut-1-yl)guanine, known as BRL 39123 or penciclovir, its salts and esters thereof and its use in the treatment of herpesvirus infections. Topical administration is disclosed as a suitable route. Hereinafter, references to penciclovir include salts and esters thereof.

It is important with a topical formulation of an antiherpesvirus drug that the quantity of drug released from the formulation is sufficient to exert a significant antiviral effect and that the drug rapidly reaches its site of action within the skin.

Rapid penetration is important, since in most cases, major virus induced epidermal pathology occurs within the first 24 hours post infection. Once the infection is established and the stratum corneum has been eroded, the rapid ingress of host resistant factors could make chemotherapy of questionable value.

WO 91/11187 (Beecham Group plc.) discloses an oil-in-water topical pharmaceutical formulation or an aqueous formulation for the treatment of virus infections of the skin or mucosa, comprising at least 30% of propylene glycol and solubilised penciclovir.

A new antiviral topical formulation has now been discovered, having improved properties as compared with conventional formulations.

Accordingly, the present invention provides an oil-in-water topical pharmaceutical formulation or an aqueous formulation for the treatment of virus infections of the skin or mucosa, comprising at least 30% w/w of propylene glycol, at least 1 to 10% w/w penciclovir and 0.5 to 1% w/w Cetomacrogol 1000.

Such a topical formulation may contain
from 30% to 60% w/w of propylene glycol and from 15% w/w water (up to 50% when there is an oil phase).

In a preferred aspect the formulation comprises from 1% to 10% w/w penciclovir, from 30% to 50% w/w of propylene glycol, from 20% w/w water (up to 40%, when there is an oil phase). Examples of suitable formulations comprise from 2% to 5% w/w penciclovir, from 35% to 45% w/w of propylene glycol, from 25% to 40% w/w water together with an oil phase. A preferred formulation, however, comprises 1% to 5% penciclovir. The formulation should preferably contain about 40% w/w of propylene glycol.

The amount of penciclovir present in the formulation should be at least sufficient to maintain an antivirally effective concentration at the site of infection between applications without showing signs of toxicity. The optimum concentration of penciclovir will depend on its solubility in the vehicle. Penciclovir may be included in the formulation at a level exceeding its solubility in order to provide a reservoir and to maintain the antiviral agent at a saturated concentration within the vehicle. One suitable amount in the above preferred formulation is up to 10% w/w, such as 2-8%, for example 5%. A preferred amount is less than 5%, such as 1 - 2%, for example 1%.

The water used in the formulation is preferably purified water, purified that is by distillation by means of ion exchange or other appropriate method, such as reverse osmosis.

The oil phase of the emulsions of this invention may be constituted from known ingredients in a known manner. While the phase may comprise merely an emulsifier (otherwise known as an emulgent), it is desirably comprised of a mixture of at least one emulsifier with one or more excipients including oils, fats and/or waxes, together with optional film formers and stabilisers as well as thickening and bodying agents. Preferably, as explained in more detail below, an additional hydrophilic emulsifier is included together with a lipohilic emulsifier which acts as a stabiliser. It is also preferred to include both an oil and a fat. Together, the emulsifier(s) make up the so-called emulsifying wax, and the wax together with the oil and/or fat make up the so called emulsifying ointment base which forms the oil dispersed phase of the emulsions.

Oil-in-water topical formulations may be formulated in a number of ways, all of which depend primarily on the alignment of the emulgent or emulsifying agent and emulsion stabiliser at the oil/water interface, with the non-polar or lipophilic groups soluble in the oil phase and the polar or hydrophilic groups in the aqueous or continuous phase. Thus the more polar hydrophilic emulgents result in oil-in-water emulsions. This principle has been systemised in the idea of a 'hydrophilic-lipophilic balance' (H.L.B.) Griffen, W. C. **J. Soc. Cosmet. Chem.,** 1954, 5, 249 and the various emulgents have been allocated H.L.B. numbers from which their behaviour with constituents of the aqueous and oil phases (to which are applied theoretical required H.L.B. figures) may be predicted.

The emulsion stabiliser for use in the formulation of the present invention is *Cetomacrogol 1000* (Polyethylene Glycol 1000 Monocetyl Ether) present in a proportion of 0.5 to 1% by weight, such as 0.7 to 1.0%, preferably 0.9% to 1.0%, the optimum concentration being 0.94%.

The choice of suitable oils or fats for the formulation is based on achieving the desired cosmetic properties. Thus the cream should preferably be non-greasy, non-staining and washable product with suitable consistency to avoid leakage from tubes or other containers. Lipophilic substances with relatively high melting points, such as beeswax, partial glycerides of capric and caprylic acids, or silicone oil, white soft paraffin and/or liquid paraffin or other mineral or vegetable oils are suitable. Straight or branched chain, mono- or dibasic alkyl esters such as di-isopropyladipate, isocetyl stearate, propylene glycol diester of coconut fatty acids, isopropyl myristate, decyl oleate, isopropyl palmitate, butyl stearate, 2-ethylhexyl palmitate or a mixed ester of 2-ethyl hexanoic acid with a blend of cetyl or stearyl alcohols known as Crodamol CAP may also be used.

As well as creams, the aqueous/oil-in-water formulation may be a lotion, skin paint, gel, spray, aerosol, liniment or gel stick, which are formulated as known in the art, for example as described in standard text books of pharmaceutics and cosmetics, such as Harry's Cosmeticology published by Leonard Hill Books, the British Pharmacopoeia, USP twenty-first revision (USP XXI) (1984), distributed by Mack Publishing Company.

The product may or may not be sterile, with adequate preservative capacity for single or multi-dose purposes.

In this description, the following terms are employed:

**Aerosol** - Pharmaceutical aerosols are products that are packaged under pressure and contain therapeutically active ingredients that are released upon activation of an appropriate valve system. The term 'aerosol' has been used to refer to the fine mist of spray that is emitted from a pressurized container containing an active ingredient and a propellant. However, the term has been broadly applied to include all self-contained pressurized products, some of which deliver foams or semisolid fluids. Accordingly, unless indicated otherwise, a reference herein to an aerosol formulation of the present invention should be understood to include pharmaceutical compositions for topical use comprising a pharmaceutically acceptable carrier which includes a propellant, said compositions being adapted for use in a pressurized container that dispenses the composition as a spray, foam or semisolid liquid.

An aerosol generally comprises a container, a propellant, a concentrate containing the active ingredient, a valve (which may be a metered valve), and an actuator. The nature of these components determines characteristics such as delivery rate, foam density, and fluid viscosity. Aerosols may be two-phase (gas and liquid) or three-phase (gas, liquid, and solid or liquid formulations. A two-phase formulation consists of a solution of active ingredients in liquidified propellant and the vaporized propellant. The solvent may be the propellant or a mixture of the propellant and co-solvents such as alcohol and polyethylene glycols which are often used to enhance the solubility of the active ingredients. Three-phase formulations consist of a suspension or emulsion of the active ingredient(s) in addition to the vaporized propellants. A suspension consists of the active ingredient(s) dispersed in the propellant system with the aid of suitable excipients such as wetting agents and/or solid carriers such as talc or colloidal silicas. A foam formulation is generally an emulsion containing one or more active ingredients, surfactants, aqueous or nonaqueous liquids, and the propellants. If the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. [See **The United States Pharmacopeia, XXI** ('USP') at 1334].

**Gels** - Gels are semisolid systems consisting either of suspensions made up of small inorganic particles or of large organic molecules interpenetrated by a liquid. Where the gel consists of a network of small discrete particles, the gel is classified as a two-phase system. In a two-phase gel, if the particle size of the dispersed phase is relatively large, the gel is sometimes referred to as a magma. Both gels and magmas may be thixotropic, forming semisolids on standing and becoming liquid on agitation.

Single-phase gels consist of organic macromolecules uniformly distributed throughout a liquid so that no apparent boundaries exist between the dispersed macromolecules and the liquid. Single-phase gels may be made from synthetic macromolecules (e.g. *Carbomer*)* or from natural gums (e.g. *Tragacanth*). The latter preparations are also called mucilages. Although single-phase gels are commonly aqueous, alcohols and oils may also be used as the continuous phase. For example, mineral oil can be combined with a polyethylene resin to form a gel which may be used as an oleaginous ointment base [see **USP, supra** at 1336].
*Excipients that are italicised in this discussion are classified as pharmacopoeial preparations (USP or BP).

Lotion - Preferred lotions include fluid or thixotropic emulsions intended for external application to the body. These lotions are emulsions of the oil-in-water type stabilized by a surface-active agent. They may separate or stratify on long standing, and should be well shaken before each use. Adequate preservation against microbial contamination is required [see **USP, supra** at 1337].

**Gel stick** - The definition of gel sticks set forth in **Harry's Cosmeticology**, 6th Edition, at 740, is hereby incorporated herein by reference.

**Liniment** - Liniments are solutions or mixtures of various substances in oil, alcoholic solutions of soap, or emulsions, as in the present invention. They are intended for external application and are usually applied with friction and rubbing of skin, the oil or soap base providing for ease of application and massage.

**Spray** - As used in this description, spray formulations are aqueous solutions of various drugs which are applied topically from a container having a spray means (e.g., an atomizer or nebulizer).

The present invention further provides a method for the preparation of a topical pharmaceutical formulation, as hereinbefore defined, which comprises mixing the combination of penciclovir, propylene glycol, Cetomacrogol 1000 and water, optionally with oil phase.

The manner of formulating an emulsion will of course vary according to the amount and nature of the constituents, but nevertheless follows known techniques in emulsion technology (see The Pharmaceutical Codex, London, The Pharmaceutical Press, 1979). In a preferred method penciclovir may be included in the oil phase prior to emulsification with the aqueous portion. Alternatively the penciclovir may be initially incorporated wholly in the aqueous portion where it may form a solution alone, or mixed solution/suspension, and then emulsified with the ointment base, or a part of the aqueous portion may be formulated as an emulsion, and the balance of the water, propylene glycol and penciclovir added to and dispersed into the emulsion. In using these procedures, it is preferable to heat the aqueous portion and the ointment base to about 40 to 80°C, preferably 50 to 70°C, prior to emulsification which may be achieved by vigorous agitation using for example a standard laboratory mixer. Finer dispersions of the oil phase may be obtained by homogenising or milling in a colloidal mill.

A topical formulation of the present invention may be used in the treatment or prevention of viral infections caused by herpesviruses such as herpes simplex types 1 and 2 and varicella-zoster virus.

The formulation should be applied to the affected area 2 to 6 times daily, preferably 2 or 3 times.

The following examples illustrate the invention.

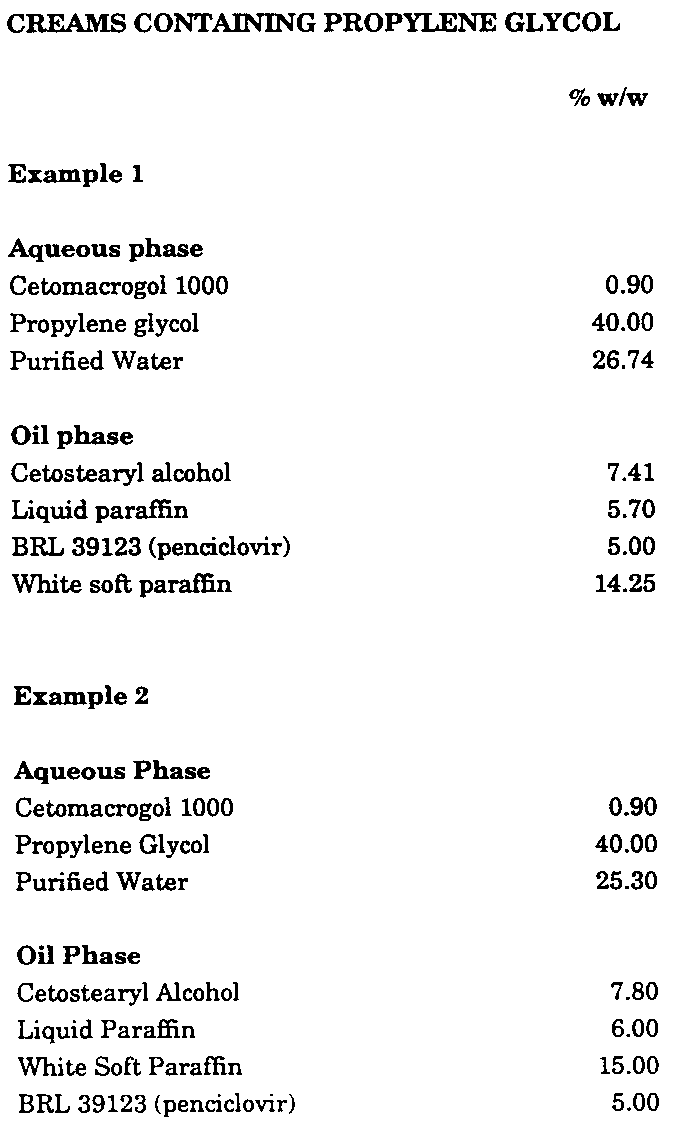

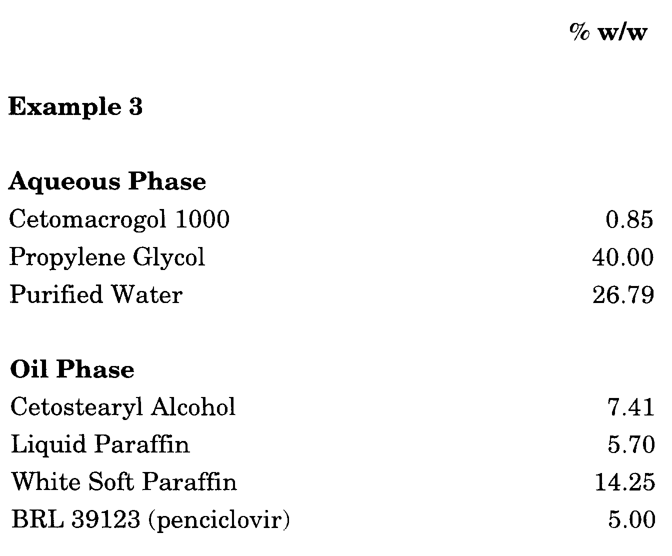

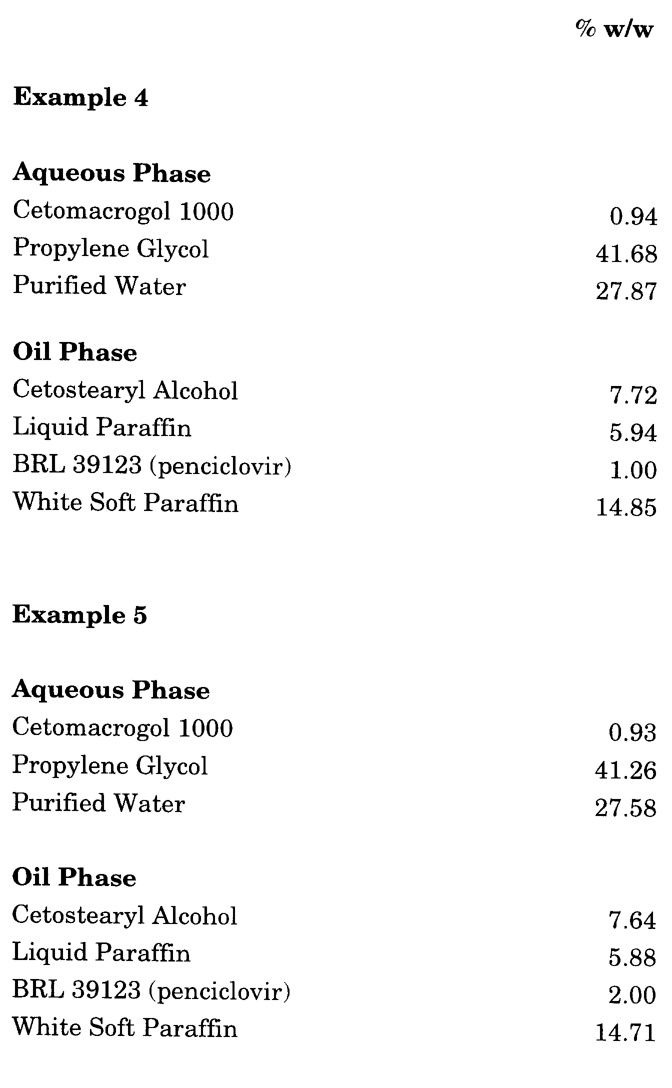

## Claims

1. An oil-in-water or an aqueous topical pharmaceutical formulation for the treatment of virus infections of the skin or mucosa, comprising at least 30% w/w propylene glycol, 1 to 10% w/w penciclovir and 0.5 to 1% w/w Cetomacrogol 1000.

2. A pharmaceutical formulation according to claim 1, comprising 30 to 60% w/w propylene glycol and at least 15% w/w water.

3. A pharmaceutical formulation according to claim 2, comprising 30 to 50% w/w propylene glycol and at least 20% w/w water.

4. A pharmaceutical formulation according to claim 3, comprising 1 to 5% w/w penciclovir, 35 to 45% w/w propylene glycol and 25 to 40% w/w water together with an oil phase.

5. A pharmaceutical formulation according to any one of claims 1 to 4, comprising 1% w/w penciclovir.

6. A pharmaceutical formulation according to any one of claims 1 to 5, comprising 0.9 to 1.0% w/w Cetomacrogol 1000.

7. A method for the preparation of a pharmaceutical formulation according to any one of claims 1 to 6, which method comprises admixing the combination of penciclovir, propylene glycol, Cetomacrogol 1000 and water, optionally with an oil phase.

8. A pharmaceutical formulation according to any one of claims 1 to 6 for use as an active therapeutic substance.

9. A pharmaceutical formulation according to any one of claims claims 1 to 6 for use in the treatment of viral infections.

10. Use of a pharmaceutical formulation according to any one of claims 1 to 6 in the manufacture of a medicament for use in the treatment of viral infections.

## Patentansprüche

1. Öl-in-Wasser- oder wäßrige topische pharmazeutische Formulierung zur Behandlung von Virusinfektionen der Haut oder der Schleimhaut, umfassend mindestens 30% (Gew./Gew.) Propylenglycol, 1 bis 10% (Gew./Gew.) Penciclovir und 0,5 bis 1% (Gew./Gew.) Cetomacrogol 1000.

2. Pharmazeutische Formulierung nach Anspruch 1, umfassend 30 bis 60% (Gew./Gew.) Propylenglycol und mindestens 15% (Gew./Gew.) Wasser.

3. Pharmazeutische Formulierung nach Anspruch 2, umfassend 30 bis 50% (Gew./Gew.) Propylglycol und mindestens 20% (Gew./Gew.) Wasser.

4. Pharmazeutische Formulierung nach Anspruch 3, umfassend 1 bis 5% (Gew./Gew.) Penciclovir, 35 bis 45% (Gew./Gew.) Propylenglycol und 25 bis 40% (Gew./Gew.) Wasser, zusammen mit einer Ölphase.

5. Pharmazeutische Formulierung nach einem der Ansprüche 1 bis 4, umfassend 1% (Gew./Gew.) Penciclovir.

6. Pharmazeutische Formulierung nach eine der Ansprüche 1 bis 5, umfassend 0,9 bis 1,0% (Gew./Gew.) Cetomacrogol 1000.

7. Verfahren zur Herstellung einer pharmazeutischen Formulierung nach einem der Ansprüche 1 bis 6, wobei das Verfahren das Mischen der Kombination aus Penciclovir, Propylenglycol, Cetomacrogol 1000 und Wasser, gegebenenfalls mit einer Ölphase, umfaßt.

8. Pharmazeutische Formulierung nach einem der Ansprüche 1 bis 6 zur Verwendung als wirksame therapeutische Substanz.

9. Pharmazeutische Formulierung nach einem der Ansprüche 1 bis 6 zur Verwendung bei der Behandlung von Virusinfektionen.

10. Verwendung einer pharmazeutisch Formulierung nach einem der Ansprüche 1 bis 6 zur Herstellung eines Medikaments zur Verwendung bei der Behandlung von Virusinfektionen.

## Revendications

1. Formulation pharmaceutique topique huile-dans-eau ou aqueuse pour le traitement d'infections virales de la peau ou des muqueuses, comprenant au moins 30 % en poids/poids de propylèneglycol, 1 à 10 % en poids/poids de penciclovir et 0,5 à 1 % en poids/poids de Cetomacrogol 1000.

2. Formulation pharmaceutique suivant la revendication 1, comprenant 30 à 60 % en poids/poids de propylèneglycol et au moins 15 % en poids/poids d'eau.

3. Formulation pharmaceutique suivant la revendication 2, comprenant 30 à 50 % en poids/poids de propylèneglycol et au moins 20 % en poids/poids d'eau.

4. Formulation pharmaceutique suivant la revendication 3, comprenant 1 à 5 % en poids/poids de penciclovir, 35 à 45 % en poids/poids de propylèneglycol et 25 à 40 % en poids/poids d'eau, conjointement avec une phase huileuse.

5. Formulation pharmaceutique suivant l'une quelconque des revendications 1 à 4, comprenant 1 % en poids/poids de penciclovir.

6. Formulation pharmaceutique suivant l'une quelconque des revendications 1 à 5, comprenant 0,9 à 1,0 % en poids/poids de Cetomacrogol 1000.

7. Procédé pour la préparation d'une formulation pharmaceutique suivant l'une quelconque des revendications 1 à 6, procédé qui comprend le mélange de l'association de penciclovir, de propylèneglycol, de Cetomacrogol 1000 et d'eau, facultativement avec une phase huileuse.

8. Formulation pharmaceutique suivant l'une quelconque des revendications 1 à 6, destinée à être utilisée comme substance thérapeutique active.

9. Formulation pharmaceutique suivant l'une quelconque des revendications 1 à 6, destinée à être utilisée dans le traitement d'infections virales.

10. Utilisation d'une formulation pharmaceutique suivant l'une quelconque des revendications 1 à 6, dans la production d'un médicament destiné à être utilisé dans le traitement d'infections virales.
